# EUROPÄISCHE PATENTSCHRIFT

(11) **EP 2 539 007 B1**
(45) Veröffentlichungstag und Bekanntmachung des Hinweises auf die Patenterteilung: **09.10.2013**
(21) Anmeldenummer: 10798734.9
(22) Anmeldetag: 16.12.2010
(51) Int. Cl.: A61M 25/01, A61B 1/005, G02B 23/24, A61B 1/008

(54) **VORRICHTUNG ZUM BEOBACHTEN UND/ODER MANIPULIEREN VON IN EINEM DURCH EINE ENGE ÖFFNUNG ZUGÄNGLICHEN HOHLRAUM ANGEORDNETEN OBJEKTEN**
DEVICE FOR MONITORING AND/OR MANIPULATING OBJECTS ARRANGED IN A CAVITY THAT CAN BE ACCESSED THROUGH A NARROW OPENING
DISPOSITIF POUR OBSERVER ET/OU MANIPULER DES OBJETS DISPOSÉS DANS UNE CAVITÉ ACCESSIBLE PAR UNE OUVERTURE ÉTROITE

(30) Priorität: 23.02.2010 DE 102010008922
(43) Veröffentlichungstag der Anmeldung: 02.01.2013
(73) Patentinhaber: Schölly Fiberoptic GmbH, 79211 Denzlingen (DE)
(72) Erfinder: SCHÖLLY, Werner, 79211 Denzlingen (DE); HOFER, Axel, 79346 Endingen (DE)
(74) Vertreter: Börjes-Pestalozza, Heinrich
(86) Internationale Anmeldenummer: PCT/EP2010/007710
(87) Internationale Veröffentlichungsnummer: WO 2011/103903

(56) Entgegenhaltungen:
- EP-A1- 1 915 950
- EP-A2- 2 005 896
- WO-A2-2008/101228
- US-A1- 2007 282 371
- US-B1- 6 569 105

## Beschreibung

Die Erfindung betrifft eine Vorrichtung zum Beobachten und/oder Manipulieren von in einem durch eine enge Öffnung zugänglichen Hohlraum angeordneten Objekten, insbesondere für den Einsatz in der minimalinvasiven Medizin, mit einem rohr- oder schlauchförmigen Einführungsteil, wobei an dem Einführungsteil ein flexibler Abschnitt ausgebildet ist und in dem Einführungsteil wenigstens ein Bowdenzug zur Ansteuerung des flexiblen Abschnitts angeordnet ist, wobei in dem Einführungsteil ein Führungskanal ausgebildet ist, welcher zur Aufnahme optischer, mechanischer und/oder elektrischer Verbindungsmittel für die Beobachtung und/oder Manipulation eingerichtet ist, und wobei ein Stellelement ausgebildet ist, welches zur Ansteuerung des flexiblen Abschnitts mit dem Bowdenzug verbunden ist. Hierbei ist das distale Ende dasjenige Ende, welches in Gebrauchsstellung entfernt von einem Benutzer angeordnet ist, während das proximale Ende dasjenige Ende ist, welches in Gebrauchsstellung nahe zu dem Benutzer angeordnet ist.

Derartige Vorrichtungen sind bekannt und haben sich bewährt.

Aus der US 6569105 B1 ist eine drehbare und biegbare Biopsie-Pinzette bekannt, bei welcher ein Stellelement vollständig und unlösbar in einer Gelenkpfanne eingeschlossen ist.

Aus der US 2007/0282371 A1 ist eine Vorrichtung der eingangs beschriebenen Art bekannt, bei welcher ein Stellelement durch eine Ringanordnung gebildet ist, welche zur Ansteuerung eines flexiblen Abschnitts über einen Bowdenzug dient.

Aus der WO 2008/101228 A2 ist ein robotisches medizinisches Instrumentensystem bekannt, bei welchem ein kugelförmiges Abstandelement am distalen Ende eines Einführungsteils angeordnet ist. Dieses Abstandselement bildet ein flexibles Gelenk zwischen beweglichen Teilen und ist nicht an der Ansteuerung eines flexiblen Abschnitts beteiligt.

Aus der EP 1915950 A1 ist eine Vorrichtung zum Klebstoffauftrag bekannt, bei welcher ein Aufnahmeraum mit einer Spitze und somit mit einem dazwischen liegenden Schaft zumindest über eine durchgängige Leitung und Steuerkabel fest und unlösbar verbunden ist.

Aus der EP 2005896 A2 ist ein chirurgisches Instrument mit einem integrierten Heftapparat bekannt, bei welcher ein in einer distalen Spitze angeordneter Heftapparat fest und unlösbar mit der zugehörigen Betätigungsvorrichtung im proximalen Handgriff verbunden ist.

Der Erfindung liegt die Aufgabe zugrunde, eine Vorrichtung der eingangs genannten Art zu schaffen, welche am proximalen Ende des Einführungsteils ein möglichst geringes Baumaß aufweist.

Zur Lösung der Aufgabe sind erfindungsgemäß bei einer Vorrichtung der eingangs genannten Art die Merkmale von Anspruch 1 oder von Anspruch 2 vorgesehen. Insbesondere wird somit vorgeschlagen, dass das
Stellelement an einem proximalen Ende des Einführungsteils angeordnet ist, dass am proximalen Ende des Einführungsteils ein Gelenk mit einer Gelenkpfanne und einem korrespondierenden Gelenkhöcker ausgebildet ist, dass das Stellelement in dem Gelenk gelagert ist und dass das Stellelement eine Durchführung aufweist, welche Durchführung zur Aufnahme der optischen, mechanischen und/oder elektrischen Verbindungsmittel eingerichtet ist und welche Durchführung in Gebrauchsstellung des Stellelements in das proximale Ende des Führungskanals mündet. Das Einführungsteil ist hierbei durch die Öffnung des Hohlraums einführbar, und an dem distalen Ende des Einführungsteils können Beobachtungs- und/oder Manipulationseinrichtungen vorgesehen sein, welche mit den Verbindungsmitteln verbunden sind. Die Erfindung macht sich die überraschende Erkenntnis zunutze, dass durch die Ausbildung eines Gelenks mit Gelenkpfanne und Gelenkhöcker anstatt der bekannten Drehgelenke das Innere des Stellelements zur Durchführung der Verbindungsleitungen nutzbar wird. Somit muss die Verbindungsleitung nicht um das Stellelement herum geführt werden, wodurch Baumraum eingespart wird. Von Vorteil ist weiter, dass die Durchführung, insbesondere wenn sie durch einen Drehpunkt des Gelenks verläuft, bei einer Betätigungs- oder Verstellbewegung des Stellelements ihre Ausrichtung und Position nur geringfügig ändert. Somit wird zusätzlich erreicht, dass die in der Durchführung und dem Führungskanal angeordneten Verbindungsmitteln, also mechanische, optische und/oder elektrische Verbindungsleitungen, durch die Verstellbewegungen des Stellelements möglichst wenig beeinträchtigt werden. Die Verbindungsleitungen werden somit entlastet.

Die Durchführung kann als Loch, Bohrung, Kanal oder auf sonstige Weise ausgeführt sein.

Vorzugsweise weist die Durchführung an ihren beiden Enden jeweils eine Mündung auf und verlängert somit den Führungskanal. Hierbei kann vorgesehen sein, dass die vom Führungskanal abgewandte Mündung in Gebrauchsstellung in einen sekundären Führungskanal mündet.

Eine Lösung von eigenständiger Bedeutung kann bei einer Vorrichtung der eingangs genannten Art vorsehen, dass an einem proximalen Ende des Einführungsteils eine Handhabe für das Einführungsteil angeordnet ist, dass eine Betätigungseinrichtung zur manuellen Betätigung des Stellelements vorgesehen ist und dass die Betätigungseinrichtung mit der Handhabe über eine flexible Ansteuerungsleitung verbunden ist. Hierbei kann zusätzlich vorgesehen sein, dass am proximalen Ende des Einführungsteils ein Gelenk mit einer Gelenkpfanne und einem korrespondierenden Gelenkhöcker ausgebildet ist, dass das Stellelement in dem Gelenk gelagert ist und dass das Stellelement eine Durchführung aufweist, welche Durchführung zur Aufnahme der optischen, mechanischen und/oder elektrischen Verbindungsmittel eingerichtet ist und welche Durchführung in Gebrauchsstellung des Stellelements in das proximale Ende des Führungskanals mündet. Von Vorteil ist dabei, dass durch die flexible Ansteuerungsleitung die Betätigungseinrichtung entfernt von dem proximalen Ende des Einführungsteils angeordnet werden kann. Somit ist das erforderliche Baumaß direkt am proximalen Ende des Einführungsteils verringert. Zusätzlich wird das Einführungsteil bei Betätigung des flexiblen Abschnitts am Betätigungsteil entlastet, weil die flexible Ansteuerungsleitung eine mechanische Entkopplung zwischen Betätigungseinrichtung und Einführungsteil gegenüber ungewollten Reaktionsbewegungen während der Betätigung bewirkt.

Zur Bildung des Gelenks kann vorgesehen sein, dass die Gelenkpfanne an dem Stellelement und der Gelenkhöcker an dem proximalen Ende des Einführungsteils ausgebildet sind. Alternativ kann umgekehrt vorgesehen sein, dass die Gelenkpfanne an dem proximalen Ende des Einführungsteils und der Gelenkhöcker an dem Stellelement ausgebildet ist.

Hierbei können die Gelenkpfanne beziehungsweise der Gelenkhöcker eine Berührfläche aufweisen, welche einen Abschnitt einer zylindrischen Fläche beschreibt. Somit kann das Stellelement für eine Zwei-Wege-Ansteuerung ausgeführt werden. Es kann auch vorgesehen sein, dass die Berührfläche eine sphärische oder ellipsoidale Fläche beschreibt. Hierdurch wird eine Vier-Wege-Ansteuerung des Stellelements ermöglicht.

Zur manuellen Betätigung des Stellelements kann eine Betätigungseinrichtung vorgesehen sein.

Um eine Entlastung des Einführungsteils während der manuellen Betätigung des Stellelements zu erreichen, kann vorgesehen sein, dass die Betätigungseinrichtung über eine flexible Ansteuerungsleitung mit dem Stellelement verbunden ist. Die Verbindung kann direkt oder indirekt über eine Kupplung hergestellt sein. Somit kann vermieden werden, dass Gegenkräfte, die bei der manuellen Betätigung der Betätigungseinrichtung ungewollt erzeugt werden, auf das in die enge Öffnung bei Gebrauch eingeführte Einführungsteil übertragen werden.

Es kann vorgesehen sein, dass an dem Stellelement eine Kupplungsstelle zur formschlüssigen, vorzugsweise lösbaren, Verbindung mit einem Gegenkupplungsstück ausgebildet ist. Somit kann das Einführungsteil zum Gebrauch auf einfache Weise mit der übrigen Vorrichtung verbunden und sogar nach Gebrauch von dieser entfernt werden, was besonders dann günstig ist, wenn ein steriles bzw. sterilisiertes Einführungsteil im Gebrauch erforderlich ist.

Eine in Gebrauchsstellung im Bereich der Öffnung besonders platzsparende Vorrichtung kann vorsehen, dass das Stellelement in einer an dem proximalen Ende des Einführungsteils ausgebildeten Handhabe angeordnete ist.

Günstige Gebrauchseigenschaften ergeben sich, wenn der wenigstens eine Bowdenzug und das Stellelement und/oder die Betätigungseinrichtung zur Zwei-Wege- oder Vier-Wege-Ansteuerung des flexiblen Abschnitts eingerichtet sind. Hierzu sind in dem Einführungsteil zwei bzw. vier Bowdenzüge in an sich bekannter Weise angeordnet, und die Betätigungseinrichtung kann die erforderliche Anzahl von Hebeln und dergleichen aufweisen.

Um eine definierte Ausgangslage des flexiblen Abschnitts und/oder eine einfache Verbindbarkeit mit einem Gegenkupplungsstück zu erreichen, kann eine Rückstelleinrichtung vorgesehen sein, welche das Stellelement bei fehlender Betätigung in eine Normallage bringt. Beispielsweise kann die Rückstelleinrichtung durch eine Feder oder ein elastisches Element gebildet sein.

Bei einer Ausgestaltung der Erfindung kann vorgesehen sein, dass die Handhabe mit einer Markierung versehen ist, welche die Orientierung des Einführungsteils in Bezug auf Drehungen um dessen Verlaufsrichtung anzeigt. Somit kann ein Benutzer der Vorrichtung auf einfache Weise ohne Sichtkontrolle auf den flexiblen Abschnitt die Ausrichtung des distalen Endes des Einführungsteils im Hohlraum kontrollieren und manipulieren.

Zur Bildung einer flexiblen Ansteuerungsleitung kann vorgesehen sein, dass die Betätigungseinrichtung mit dem Stellelement über wenigstens einen sekundären Bowdenzug verbunden ist. Die Verbindung kann indirekt über eine Kupplung oder direkt hergestellt sein. Vorzugsweise sind diese sekundären Bowdenzüge ebenfalls zur Zwei-Wege- oder Vier-Wege-Ansteuerung eingerichtet.

Hierbei kann vorgesehen sein, dass der wenigstens eine sekundäre Bowdenzug der Betätigungseinrichtung an dem Stellelement oder an einem Gegenkupplungsstück in wenigstens einem sekundären Angriffspunkt angreift, dessen radialer Abstand von einem Drehpunkt des Gelenks verschieden ist von dem radialen Abstand eines primären Angriffspunkts von dem Drehpunkt des Gelenks, an welchem primären Angriffspunkt der in dem Einführungsteil angeordnete wenigstens eine Bowdenzug an dem Stellelement angreift. Von Vorteil ist dabei, dass durch die unterschiedlichen radialen Abstände Übersetzungsverhältnisse zwischen den Stellwegen der primären und sekundären Bowdenzüge ausgebildet werden können.

Besonders günstig ist es, wenn das Gegenkupplungsstück in einem zweiten Gelenk gelagert ist, welches eine Gelenkpfanne und einen korrespondierenden Gelenkhöcker aufweist. Von Vorteil ist dabei, dass das Gegenkupplungsstück auf einfache Weise die Stellbewegungen des Stellelements mitvollziehen kann, wenn es in das Stellelement eingekuppelt ist.

Hierbei kann vorgesehen sein, dass die sekundären Bowdenzüge mit dem Gegenkupplungsstück fest verbunden sind. Somit können die sekundären Bowdenzüge durch Einkuppeln des Gegenkupplungsstücks in das Stellelement auf einfache Weise mit dem Stellelement verbunden werden.

Es kann vorgesehen sein, dass die Gelenkpfanne und/oder der Gelenkhöcker des zweiten Gelenks eine sekundäre Berührfläche aufweist/aufweisen, welche sekundäre Berührfläche einen Abschnitt einer zylindrischen, sphärischen oder ellipsoidalen Fläche beschreibt. Hierbei kann vorgesehen sein, dass ein Krümmungsradius dieser sekundären Berührfläche gleich ist dem entsprechenden Krümmungsradius der Berührfläche des ersten Gelenks, oder es kann zur Erreichung eines Übersetzungsverhältnisses der Stellwege vorgesehen sein, dass ein Krümmungsradius dieser Fläche verschieden von einem Krümmungsradius der Berührfläche des ersten Gelenks ist.

Eine möglichst geringfügige Beeinträchtigung von in dem Führungskanal und der Durchführung angeordneten Verbindungsleitungen bei Stellbewegungen des Stellelements kann erreicht werden, wenn die Durchführung durch einen Gelenkdrehpunkt des ersten Gelenkes geführt ist.

Gegebenenfalls kann vorgesehen sein, dass das Gegenkupplungsstück ebenfalls eine Durchführung aufweist, welche in gekuppelter Position die Durchführung des Stellelements in proximaler Richtung fortsetzt und gegebenenfalls ebenfalls in einen Führungskanal mündet.

Die Erfindung wird nun anhand von Ausführungsbeispielen beschrieben, ist jedoch nicht auf diese Ausführungsbeispiele beschränkt. Weitere Ausführungsbeispiele ergeben sich durch Kombination einzelner oder mehrerer Merkmale der Ansprüche untereinander und/oder mit einzelnen oder mehreren Merkmalen der Ausführungsbeispiele.

Es zeigt in teilweise schematisierter Darstellung
- Fig. 1: eine erfindungsgemäße Vorrichtung zum Beobachten und/oder Manipulieren von in einem durch eine enge Öffnung zugänglichen Hohlraum angeordneten Objekten in einer dreidimensionalen Schrägansicht,
- Fig. 2: ein erfindungsgemäß ausgestaltetes Einführungsteil in dreidimensionaler Schrägansicht,
- Fig. 3: das Einführungsteil gemäß Figur 2 in einer Schnittdarstellung,
- Fig. 4: ein weiteres erfindungsgemäß ausgestaltetes Einführungsteil in einer dreidimensionalen Schrägansicht,
- Fig. 5: das Einführungsteil gemäß Figur 4 in einer Schnittansicht,
- Fig. 6: eine Detailansicht des Einführungsteils aus Figur 3 und
- Fig. 7: eine Detailansicht des Einführungsteils aus Figur 5.

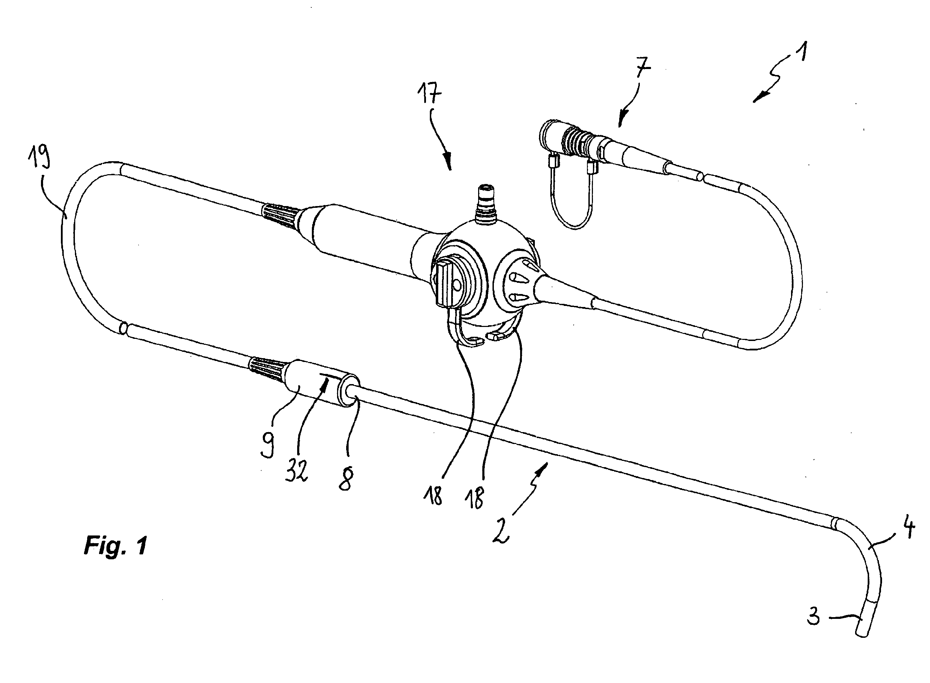

Figur 1 zeigt eine im Ganzen mit 1 bezeichnete Vorrichtung zum Beobachten und/oder Manipulieren von in einem durch eine enge Öffnung zugänglichen Hohlraum angeordneten Objekten.

Die Vorrichtung 1 hat ein Einführungsteil 2, welches rohrförmig ausgebildet ist, so dass es durch die enge Öffnung in den Hohlraum eingeführt werden kann.

Im Bereich des distalen Endes 3 des Einführungsteils 2 ist an dem Einführungsteil 2 ein flexibler Abschnitt 4 ausgebildet, durch welchen das distale Ende 3 mit dem übrigen Einführungsteil 2 beweglich verbunden ist.

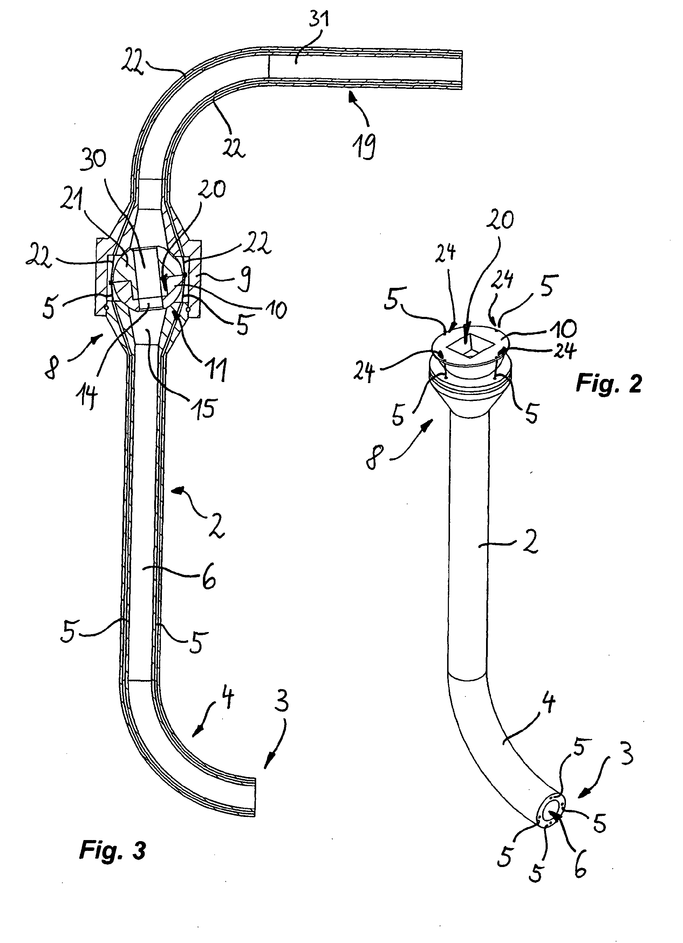

Die Form, insbesondere Biegung, des flexiblen Abschnitts 4 und damit die Position des distalen Endes 3 kann über in Figur 3 ersichtliche Seil- oder Bowdenzüge 5 gesteuert werden, welche in an sich bekannter Weise am distalen Ende 3 befestigt und in dem Einführungsteil 2 geführt sind.

Hierbei bewirkt das in Figur 3 ersichtliche Paar von Bowdenzügen 5 eine Zwei-Wege-Ansteuerung des flexiblen Abschnitts 4, und die in Figur 2 ersichtliche Kreuzanordnung von vier Bowdenzügen 5 eine Vier-Wege-Ansteuerung.

Im Inneren des Einführungsteils 2 verläuft ein in Figur 3 ersichtlicher Führungskanal 6, welcher das distale Ende 3 mit einem Anschlussstück 7 der Vorrichtung 1 verbindet, so dass das Innere des Hohlraums über das eingeführte Einführungsteil 2 und dessen Führungskanal 6 von dem Anschlussstück 7 her zugänglich ist. Hierbei kann der flexible Abschnitt 4 so gebogen werden, dass das distale Ende 3 in eine gewünschte Richtung zeigt.

Durch den Führungskanal 6 können somit nicht weiter gezeigte optische, mechanische und/oder elektrische Verbindungsmittel in den zu untersuchenden Hohlraum eingeführt werden.

An dem proximalen Ende 8 des Einführungsteils 2 ist eine Handhabe 9 ausgebildet, mit welcher das Einführungsteil 2 dirigiert werden kann.

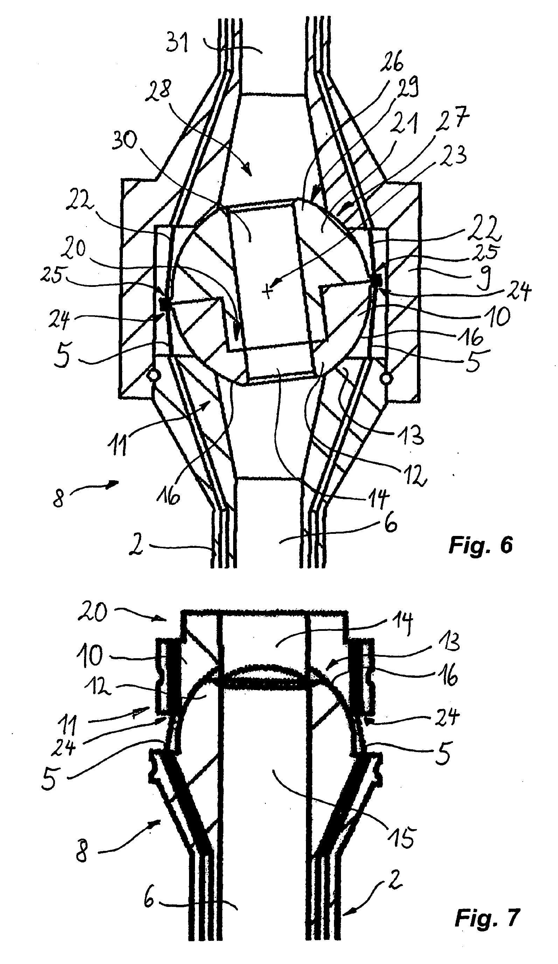

Im Inneren der Handhabe 9 ist am proximalen Ende 8 des Einführungsteils 2 ein Stellelement 10 angeordnet, wie in Figur 3 und der Detaildarstellung in Figur 6 näher ersichtlich ist.

Das Stellelement 10 und das distale Ende 8 des Einführungsteils 2 bilden ein Gelenk 11, wobei an dem Stellelement 10 ein Gelenkhöcker 12 und an dem distalen Ende 8 eine korrespondierende Gelenkpfanne 13 ausgebildet ist. Der Gelenkhöcker 12 greift in die Gelenkpfanne 13 und ist in dieser gelagert.

In dem Stellelement 10 ist eine Durchführung 14 als Bohrung ausgebildet, so dass der Führungskanal 6 von dem Anschlussstück 7 durch das Stellelement 10 hindurch zugänglich ist, da die Durchführung 14 in Gebrauchsstellung in das proximale Ende 15 des Führungskanals 6 mündet.

Um eine gleitende Lagerung des Stellelements 10 in der Gelenkpfanne 13 zu ermöglichen, ist an dem Gelenkhöcker 12 eine sphärische Berührfläche 16 ausgebildet, durch welche der Gelenkhöcker 12 eine kugelige Außenform erhält.

Da die Bowdenzüge 5 proximal an dem Stellelement 10 angreifen, kann die Biegung des flexiblen Abschnitts 4 durch die Stellung des Stellelements 10 in der Gelenkpfanne 13 variiert werden.

Zur manuellen Betätigung des Stellelements 10 ist an der Vorrichtung 1 eine Betätigungseinrichtung 17 mit Betätigungshebeln 18 vorgesehen, welche über eine flexible Ansteuerungsleitung 19 mit der Handhabe 9 und dem Stellelement 10 verbunden ist.

Hierbei kann das Einführungsteil 2 mit der flexiblen Ansteuerungsleitung 19 verbunden werden, indem das proximale Ende 8 in die Handhabe 9 eingesetzt und mit dieser verrastet wird.

Zur Übertragung der Steuerungsbewegung von der Betätigungseinrichtung 17 auf das Stellelement 10 und damit weiter auf den flexiblen Abschnitt 4 ist an dem Stellelement 10 eine Kupplungsstelle 20 ausgebildet, in welche im Gebrauch ein Gegenkupplungsstück 21 formschlüssig und lösbar eingreift.

Somit wird eine Verstellbewegung des Gegenkupplungsstücks 21 auf das Stellelement 10 und weiter auf den flexiblen Abschnitt 4 übertragen.

Wie in Figur 6 näher ersichtlich ist, greifen an dem Gegenkupplungsstück 21 sekundäre Bowdenzüge 22 an, welche über die Betätigungshebel 18 in Vier-Wege-Ansteuerung angesteuert werden können.

Durch die formschlüssige Verbindung zwischen Stellelement 10 und Gegenkupplungsstück 21 sind diese sekundären Bowdenzüge 22 mit dem Stellelement 10 verbunden und übertragen somit die manuelle Betätigung durch die Betätigungseinrichtung 17. Beim Lösen der Verbindung zwischen Stellelement 10 und Gegenkupplungsstück 21 bleiben die sekundären Bowdenzüge 22 mit dem Gegenkupplungsstück 21 verbunden, während die Bowdenzüge 5 mit dem Stellelement 10 verbunden bleiben.

Durch die kugelige oder sphärische Ausbildung der Berührungsfläche 16 weist das Gelenk 11 einen Drehpunkt 23 auf, um welchen sich das Stellelement 10 bei Betätigung dreht.

Die primären Bowdenzüge 5 des Einführungsteils 2 greifen an dem Stellelement 10 in primären Angriffspunkten 24 an, deren radialer Abstand zu dem Drehpunkt 23 gleich ist zu dem radialen Abstand der sekundären Angriffspunkte 25 der sekundären Bowdenzüge 22 an dem Kupplungsgegenstück 21.

Bei dem Ausführungsbeispiel sind die Angriffspunkte 24, 25 sogar an einander entsprechenden, gegenüberliegenden Stellen des Stellelements 10 beziehungsweise des Gegenkupplungsstücks 21 ausgebildet.

Durch die gleichen radialen Abstände werden die Stellbewegungen der sekundären Bowdenzüge 22 ohne Veränderung in entsprechende Stellbewegungen der (primären) Bowdenzüge 5 umgesetzt.

Das Gegenkupplungsstück 21 bildet einen Gelenkhöcker 26, welcher mit einer korrespondierenden Gelenkpfanne 27 der Handhabe 9 ein zweites Gelenk 28 bildet. Ähnlich der Situation bei dem Gelenk 11 ist hierbei an dem Gelenkhöcker 26 eine sphärische Berührfläche 29 ausgebildet, welche in der Gelenkpfanne 27 gleitet.

Die Berührfläche 29 und 16 ergänzen sich zu einer Kugeloberfläche, so dass der Drehpunkt des zweiten Gelenks 28 mit dem Drehpunkt 23 des ersten Gelenks 11 zusammenfällt. Hierdurch weisen die Berührflächen 16, 29 auch übereinstimmende Krümmungsradien auf.

In dem Gegenkupplungsstück 21 ist ebenfalls eine Durchführung 30 ausgebildet, welche die Durchführung 14 des Stellelements 10 fortsetzt.

Die gemeinsame Durchführung 14, 30 ist durch den Drehpunkt 23 des Gelenks 11 geführt und verbindet den Führungskanal 6 mit einem sekundären Führungskanal 31 in der flexiblen Ansteuerungsleitung 19.

Der sekundäre Führungskanal 31 ist weiter zum Anschlussstück 7 geführt und mündet dort nach draußen.

Das aus der Handhabe 5 herausnehmbare Einführungsteil 2 ist aus einem sterilisierbaren Material gefertigt.

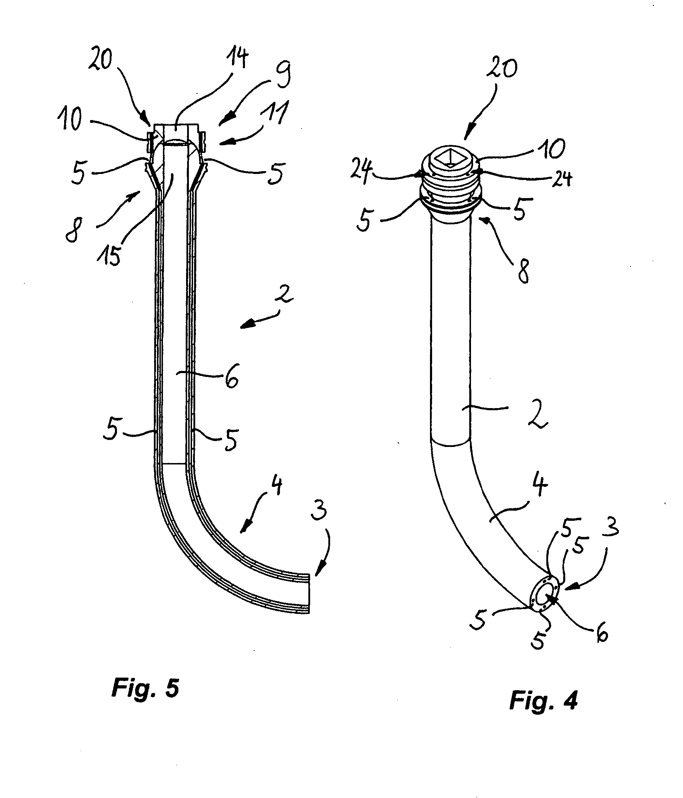

Figur 4, 5 und 7 zeigen ein weiteres erfindungsgemäß ausgestaltetes Einführungsteil 2, welches statt des Einführungsteils 2 aus Figur 2, 3 und 6 mit der Handhabe 9 verbunden werden kann.

Bei diesem Einführungsteil 2 sind funktionell und/oder konstruktiv gleichartige Teile mit denselben Bezugszeichen bezeichnet und nicht noch einmal gesondert beschrieben.

Das Einführungsteil 2 gemäß Figur 4, 5 und 7 unterscheidet sich von dem Einführungsteil 2 gemäß Figur 2, 3 und 6 dadurch, dass der Gelenkhöcker 12 des Gelenks 11 an dem proximalen Ende 8 des Einführungsteils 2 ausgeformt ist, während das Stellelement 10 eine Gelenkpfanne 13 bildet.

Hierdurch umgreift das Stellelement 10 teilweise das proximale Ende 8, wodurch das Stellelement 10 von außen besser zugänglich ist.

Das Einführungsteil 2 gemäß Figur 4, 5 und 7 kann somit auch ohne Handhabe 9 und Betätigungseinrichtung 17 verwendet werden, indem beispielsweise ein Handgriff oder dergleichen mit der Kupplungsstelle 20 des Stellelements 10 verbunden wird.

An der Handhabe 9 der Vorrichtung 1 in Figur 1 ist weiter eine Markierung 32 angebracht, mit welcher die Orientierung des möglicherweise abgebogenen distalen Endes 3 in dem zu untersuchenden Hohlraum außerhalb der Öffnung angezeigt wird.

Bei der Vorrichtung 1 zum Beobachten und/oder Manipulieren von Objekten, welche in einem durch eine enge Öffnung zugänglichen Hohlraum angeordnet sind, ist ein rohr- oder schlauchförmiges Einführungsteil 2 vorgesehen, an welchem ein flexibler Abschnitt 4 ausgebildet ist, der über einen Bowdenzug 5 ansteuerbar ist, wobei der Bowdenzug 5 an einem Stellelement 10 angreift, welches Stellelement 10 in einem Gelenk 11 drehbar gelagert ist, das eine Gelenkpfanne 13 und einen in diese Gelenkpfanne 13 eingreifenden, korrespondierenden Gelenkhöcker 12 aufweist.

## Patentansprüche

1. Vorrichtung (1) zum Beobachten und/oder Manipulieren von in einem durch eine enge Öffnung zugänglichen Hohlraum angeordneten Objekten, insbesondere für den Einsatz in der minimalinvasiven Medizin, mit einem rohr- oder schlauchförmigen Einführungsteil (2), wobei an dem Einführungsteil (2) ein flexibler Abschnitt (4) ausgebildet ist und in dem Einführungsteil (2) wenigstens ein Bowdenzug (5) zur Ansteuerung des flexiblen Abschnitts (4) angeordnet ist, wobei in dem Einführungsteil (2) ein Führungskanal (6) ausgebildet ist, welcher zur Aufnahme optischer, mechanischer und/oder elektrischer Verbindungsmittel für die Beobachtung und/oder Manipulation eingerichtet ist, und wobei ein Stellelement (10) ausgebildet ist, welches zur Ansteuerung des flexiblen Abschnitts (4) mit dem Bowdenzug (5) verbunden ist, wobei das Stellelement (10) an einem proximalen Ende (8) des Einführungsteils (2) angeordnet ist, am proximalen Ende (8) des Einführungsteils (2) ein Gelenk (11) mit einer Gelenkpfanne (13) und einem korrespondierenden Gelenkhöcker (12) ausgebildet ist, das Stellelement (10) in dem Gelenk (11) gelagert ist und das Stellelement (10) eine Durchführung (14) aufweist, welche Durchführung (14) zur Aufnahme der optischen, mechanischen und/oder elektrischen Verbindungsmittel eingerichtet ist und welche Durchführung (14) in Gebrauchsstellung des Stellelements (10) in ein proximales Ende (15) des Führungskanals (6) mündet, **dadurch gekennzeichnet, dass** an dem Stellelement (10) eine Kupplungsstelle (20) zur formschlüssigen, lösbaren Verbindung mit einem Gegenkupplungsstück (21) ausgebildet ist, wobei eine Verstellbewegung des Gegenkupplungsstücks (21) auf das Stellelement (10) und weiter auf den flexiblen Abschnitt (4) übertragbar ist, und dass das Gegenkupplungsstück (21) in einem zweiten Gelenk (28) gelagert ist, welches eine Gelenkpfanne und einen korrespondierenden Gelenkhöcker (26) aufweist, wobei die Gelenkhöcker (12, 26) jeweils eine Berührfläche (16, 29) aufweisen, welche einen Abschnitt einer sphärischen Fläche beschreibt, wobei sich die Berührflächen (16, 29) in verbundener Position zu einer Kugeloberfläche ergänzen.

2. Vorrichtung (1) zum Beobachten und/oder Manipulieren von in einem durch eine enge Öffnung zugänglichen Hohlraum angeordneten Objekten, insbesondere für den Einsatz in der minimalinvasiven Medizin, mit einem rohr- oder schlauchförmigen Einführungsteil (2), wobei an dem Einführungsteil (2) ein flexibler Abschnitt (4) ausgebildet ist und in dem Einführungsteil (2) wenigstens ein Bowdenzug (5) zur Ansteuerung des flexiblen Abschnitts (4) angeordnet ist, wobei in dem Einführungsteil (2) ein Führungskanal (6) ausgebildet ist, welcher zur Aufnahme optischer, mechanischer und/oder elektrischer Verbindungsmittel für die Beobachtung und/oder Manipulation eingerichtet ist, und wobei ein Stellelement (10) ausgebildet ist, welches zur Ansteuerung des flexiblen Abschnitts (4) mit dem Bowdenzug (5) verbunden ist, wobei das Stellelement (10) an einem proximalen Ende (8) des Einführungsteils (2) angeordnet ist, am proximalen Ende (8) des Einführungsteils (2) ein Gelenk (11) mit einer Gelenkpfanne (13) und einem korrespondierenden Gelenkhöcker (12) ausgebildet ist, das Stellelement (10) in dem Gelenk (11) gelagert ist und das Stellelement (10) eine Durchführung (14) aufweist, welche Durchführung (14) zur Aufnahme der optischen, mechanischen und/oder elektrischen Verbindungsmittel eingerichtet ist und welche Durchführung (14) in Gebrauchsstellung des Stellelements (10) in ein proximales Ende (15) des Führungskanals (6) mündet, **dadurch gekennzeichnet, dass** an dem Stellelement (10) eine Kupplungsstelle (20) zur formschlüssigen, lösbaren Verbindung mit einem Gegenkupplungsstück (21) ausgebildet ist, wobei eine Verstellbewegung des Gegenkupplungsstücks (21) auf das Stellelement (10) und weiter auf den flexiblen Abschnitt (4) übertragbar ist, dass die Betätigungseinrichtung (17) mit dem Stellelement (10) über wenigstens einen sekundären Bowdenzug (22) verbunden ist, wobei die sekundären Bowdenzüge (22) durch die formschlüssige Verbindung zwischen dem Stellelement (10) und dem Gegenkupplungsstück (21) mit dem Stellelement (10) verbunden sind und die manuelle Betätigung durch die Betätigungseinrichtung (17) übertragen, und dass beim Lösen der Verbindung zwischen dem Stellelement (10) und dem Gegenkupplungsstück (21) die sekundären Bowdenzüge (22) mit dem Gegenkupplungsstück (21) verbunden bleiben, während die primären Bowdenzüge (5) mit dem Stellelement (10) verbunden bleiben.

3. Vorrichtung (1) nach Anspruch 1 oder 2, **dadurch gekennzeichnet, dass** an einem proximalen Ende (8) des Einführungsteils (2) eine Handhabe (9) für das Einführungsteil (2) angeordnet ist, dass eine Betätigungseinrichtung (17) zur manuellen Betätigung des Stellelements (10) vorgesehen ist und dass die Betätigungseinrichtung (17) mit der Handhabe (9) über eine flexible Ansteuerungsleitung (19) verbunden ist.

4. Vorrichtung (1) nach einem der Ansprüche 1 bis 3, **dadurch gekennzeichnet, dass** die Gelenkpfanne (13) an dem Stellelement (10) und der Gelenkhöcker (12) an dem proximalen Ende (8) des Einführungsteils (2) ausgebildet ist oder dass die Gelenkpfanne (13) an dem proximalen Ende (8) des Einführungsteils (2) und der Gelenkhöcker (12) an dem Stellelement (10) ausgebildet ist.

5. Vorrichtung (1) nach Anspruch 2 oder einem davon abhängigen Ansprüchen, **dadurch gekennzeichnet, dass** die Gelenkpfanne (13) und/oder der Gelenkhöcker (12) eine Berührfläche (16) aufweist/aufweisen, welche Berührfläche (16) einen Abschnitt einer zylindrischen, sphärischen oder ellipsoidalen Fläche beschreibt.

6. Vorrichtung (1) nach einem der Ansprüche 1 bis 5, **dadurch gekennzeichnet, dass** eine Betätigungseinrichtung (17) zur manuellen Betätigung des Stellelements (10) vorgesehen ist und/oder dass die Betätigungseinrichtung (17) über eine flexible Ansteuerungsleitung (19) mit dem Stellelement (10) verbunden ist.

7. Vorrichtung (1) nach einem der Ansprüche 1 bis 6, **dadurch gekennzeichnet, dass** das Stellelement (10) in einer an dem proximalen Ende (8) des Einführungsteils (2) ausgebildeten Handhabe (9) angeordnet ist.

8. Vorrichtung (1) nach einem der Ansprüche 1 bis 7, **dadurch gekennzeichnet, dass** der wenigstens eine Bowdenzug (5) und das Stellelement (10) und/oder die Betätigungseinrichtung (17) zur Zwei-Wege- oder Vier-Wege-Ansteuerung des flexiblen Abschnitts (4) eingerichtet sind und/oder dass eine Rückstelleinrichtung vorgesehen ist, welche das Stellelement (10) bei fehlender Betätigung in eine Normallage bringt.

9. Vorrichtung (1) nach einem der Ansprüche 1 bis 8, **dadurch gekennzeichnet, dass** die Handhabe (9) mit einer Markierung (32) versehen ist, welche die Orientierung des Einführungsteils (2) in Bezug auf Drehungen um dessen Verlaufsrichtung anzeigt.

10. Vorrichtung (1) nach Anspruch 1 oder einem davon abhängigen Ansprüchen, **dadurch gekennzeichnet, dass** die Betätigungseinrichtung (17) mit dem Stellelement (10) über wenigsten einen sekundären Bowdenzug (22) verbunden ist und/oder dass der wenigstens eine sekundäre Bowdenzug (22) der Betätigungseinrichtung (17) an dem Stellelement (10) oder an einem Gegenkupplungsstück (21) in wenigstens einem sekundären Angriffspunkt (25) angreift, dessen radialer Abstand von einem Drehpunkt (23) des Gelenks (11) verschieden ist von dem radialen Abstand eines primären Angriffspunktes (24) von dem Drehpunkt (23) des Gelenks (11), an welchem primären Angriffspunkt (24) der in dem Einführungsteil (2) angeordnete wenigstens eine Bowdenzug (5) an dem Stellelement (10) angreift.

11. Vorrichtung (1) nach Anspruch 2 oder einem davon abhängigen Ansprüchen **dadurch gekennzeichnet, dass** das Gegenkupplungsstück (21) in einem zweiten Gelenk (28) gelagert ist, welches eine Gelenkpfanne (27) und einen korrespondierenden Gelenkhöcker (26) aufweist und/oder dass die Gelenkpfanne (27) und/oder der Gelenkhöcker (26) des zweiten Gelenks (28) eine sekundäre Berührfläche (29) aufweist/aufweisen, welche sekundäre Berührfläche (29) einen Abschnitt einer zylindrischen, sphärischen oder ellipsoidalen Fläche beschreibt, insbesondere wobei ein Krümmungsradius dieser Fläche verschieden von einem Krümmungsradius der Berührfläche (16) des ersten Gelenks (11) ist.

12. Vorrichtung (1) nach einem der Ansprüche 1 bis 11, **dadurch gekennzeichnet, dass** die Durchführung (14) durch einen Drehpunkt (23) des Gelenks (11) geführt ist.

## Claims

1. Device (1) for monitoring and/or manipulating objects that are arranged in a cavity that can be accessed through a narrow opening, in particular for use in minimally invasive medicine, with a tubular or hose-shaped insertion part (2), wherein a flexible section (4) is formed on the insertion part (2), and at least one Bowden cable (5) for controlling the flexible section (4) is arranged in the insertion part (2), wherein the insertion part (2) contains a guide channel (6), which is designed to receive optical, mechanical and/or electrical connectors for the monitoring and/or manipulation, and wherein an adjusting element (10) is provided, which is connected to the Bowden cable (5) in order to control the flexible section (4), wherein the adjusting element (10) is arranged on a proximal end (8) of the insertion part (2), a joint (11) with a joint socket (13) and with a corresponding joint ball (12) is formed on the proximal end (8) of the insertion part (2), the adjusting element (10) is mounted in the joint (11), and the adjusting element (10) has a passage (14), which passage (14) is designed to receive the optical, mechanical and/or electrical connectors, and which passage (14) opens into a proximal end (15) of the guide channel (6) when the adjusting element (10) is in the position of use, **characterized in that** a coupling point (20) for form-fit and releasable connection to a mating coupling piece (21) is formed on the adjusting element (10), wherein a displacement of the mating coupling piece (21) is transferrable to the adjusting element (10) and onward to the flexible section (4), and **in that** the mating coupling piece (21) is mounted in a second joint (28), which has a joint socket and a corresponding joint ball (26), wherein the joint balls (12, 26) each have a contact surface (16, 29) that describes a portion of a spherical surface, wherein the contact surfaces (16, 29), in the connected position, complement each other to form a ball surface.

2. Device (1) for monitoring and/or manipulating objects that are arranged in a cavity that can be accessed through a narrow opening, in particular for use in minimally invasive medicine, with a tubular or hose-shaped insertion part (2), wherein a flexible section (4) is formed on the insertion part (2), and at least one Bowden cable (5) for controlling the flexible section (4) is arranged in the insertion part (2), wherein the insertion part (2) contains a guide channel (6), which is designed to receive optical, mechanical and/or electrical connectors for the monitoring and/or manipulation, and wherein an adjusting element (10) is provided, which is connected to the Bowden cable (5) in order to control the flexible section (4), wherein the adjusting element (10) is arranged on a proximal end (8) of the insertion part (2), a joint (11) with a joint socket (13) and with a corresponding joint ball (12) is formed on the proximal end (8) of the insertion part (2), the adjusting element (10) is mounted in the joint (11), and the adjusting element (10) has a passage (14), which passage (14) is designed to receive the optical, mechanical and/or electrical connectors, and which passage (14) opens into a proximal end (15) of the guide channel (6) when the adjusting element (10) is in the position of use, **characterized in that** a coupling point (20) for form-fit and releasable connection to a mating coupling piece (21) is formed on the adjusting element (10), wherein a displacement of the mating coupling piece (21) is transferrable to the adjusting element (10) and onward to the flexible section (4), and **in that** the actuating mechanism (17) is connected to the adjusting element (10) via at least one secondary Bowden cable (22), wherein the secondary Bowden cables (22) are connected to the adjusting element (10) by the form-fit connection between the adjusting element (10) and the mating coupling piece (21) and transmit the manual actuation by the actuating mechanism (17), and **in that**, when the connection between the adjusting element (10) and the mating coupling piece (21) is released, the secondary Bowden cables (22) remain connected to the mating coupling piece (21), while the primary Bowden cables (5) remain connected to the adjusting element (10).

3. Device (1) according to Claim 1 or 2, **characterized in that** a handle (9) for the insertion part (2) is arranged on a proximal end (8) of the insertion part (2), **in that** an actuating mechanism (17) for manual actuation of the adjusting element (10) is provided, and **in that** the actuating mechanism (17) is connected to the handle (9) via a flexible control line (19).

4. Device (1) according to one of Claims 1 through 3, **characterized in that** the joint socket (13) is formed on the adjusting element (10), and the joint ball (12) is formed on the proximal end (8) of the insertion part (2), or **in that** the joint socket (13) is formed on the proximal end (8) of the insertion part (2), and the joint ball (12) is formed on the adjusting element (10).

5. Device (1) according to Claim 2 or any claim dependent thereupon, **characterized in that** the joint socket (13) and/or the joint ball (12) have/has a contact surface (16), which contact surface (16) describes a portion of a cylindrical, spherical or ellipsoid surface.

6. Device (1) according to one of Claims 1 through 5, **characterized in that** an actuating mechanism (17) for manual actuation of the adjusting element (10) is provided, and/or **in that** the actuating mechanism (17) is connected to the adjusting element (10) via a flexible control line (19).

7. Device (1) according to one of Claims 1 through 6, **characterized in that** the adjusting element (10) is arranged in a handle (9) formed on the proximal end (8) of the insertion part (2).

8. Device (1) according to one of Claims 1 through 7, **characterized in that** the at least one Bowden cable (5) and the adjusting element (10) and/or the actuating mechanism (17) are designed for two-way or four-way control of the flexible section (4), and/or **in that** a restoring mechanism is provided, which brings the adjusting element (10) to a normal position in the absence of actuation.

9. Device (1) according to one of Claims 1 through 8, **characterized in that** the handle (9) is provided with a marking (32), which indicates the orientation of the insertion part (2) with respect to rotations about the direction of extension of the latter.

10. Device (1) according to Claim 1 or any claim dependent thereupon, **characterized in that** the actuating mechanism (17) is connected to the adjusting element (10) via at least one secondary Bowden cable (22), and/or **in that** the at least one secondary Bowden cable (22) of the actuating mechanism (17) engages on the adjusting element (10) or on a mating coupling piece (21) at at least one secondary engagement point (25), of which the radial distance from a rotation point (23) of the joint (11) is different than the radial distance of a primary engagement point (24) from the rotation point (23) of the joint (11), at which primary engagement point (24) the at least one Bowden cable (5) arranged in the insertion part (2) engages on the adjusting element (10).

11. Device (1) according to Claim 2 or any claim dependent thereupon, **characterized in that** the mating coupling piece (21) is mounted in a second joint (28), which has a joint socket (27) and a corresponding joint ball (26), and/or **in that** the joint socket (27) and/or the joint ball (26) of the second joint (28) have/has a secondary contact surface (29), which secondary contact surface (29) describes a portion of a cylindrical, spherical or ellipsoid surface, particularly wherein a radius of curvature of this surface is different than a radius of curvature of the contact surface (16) of the first joint (11).

12. Device (1) according to one of Claims 1 through 11, **characterized in that** the passage (14) is routed through a rotation point (23) of the joint (11).

## Revendications

1. Dispositif (1) dévolu à l'observation et/ou à la manipulation d'objets situés dans une cavité accessible par une ouverture étroite, notamment destiné à l'utilisation en médecine mini-invasive et équipé d'une partie d'insertion (2) en forme de tube ou de tuyau souple, un segment flexible (4) étant ménagé sur ladite partie d'insertion (2), et ladite partie d'insertion (2) renfermant au moins un câble Bowden (5) dédié à l'activation dudit segment flexible (4), sachant qu'un canal de guidage (6), pratiqué dans ladite partie d'insertion (2), est agencé pour recevoir des moyens de liaison optiques, mécaniques et/ou électriques affectés à l'observation et/ou à la manipulation, et sachant qu'il est ménagé un élément de réglage (10) relié audit câble Bowden (5) en vue de l'activation dudit segment flexible (4), ledit élément de réglage (10) étant disposé à une extrémité proximale (8) de la partie d'insertion (2), laquelle extrémité proximale (8) de la partie d'insertion (2) présente une articulation (11) comportant une cuvette d'articulation (13) et un mentonnet d'articulation (12) concordant, l'élément de réglage (10) étant monté dans ladite articulation (11) et ledit élément de réglage (10) étant muni d'une traversée (14), laquelle traversée (14) est agencée en vue de recevoir lesdits moyens de liaison optiques, mécaniques et/ou électriques, et laquelle traversée (14) débouche dans une extrémité proximale (15) du canal de guidage (6) en position d'utilisation de l'élément de réglage (10), **caractérisé par le fait qu'**une zone d'accouplement (20) est ménagée sur l'élément de réglage (10) en vue de la liaison libérable, par concordance de formes, avec une pièce d'accouplement complémentaire (21), un mouvement d'ajustement de ladite pièce d'accouplement complémentaire (21) pouvant être transmis à l'élément de réglage (10), et répercuté sur le segment flexible (4) ; et **par le fait que** ladite pièce d'accouplement complémentaire (21) est montée dans une seconde articulation (28) pourvue d'une cuvette d'articulation et d'un mentonnet d'articulation (26) concordant, sachant que les mentonnets d'articulation (12, 26) sont respectivement dotés d'une surface de contact (16, 29) qui matérialise une région d'une surface sphérique, les surfaces de contact (16, 29) se complétant mutuellement, à l'emplacement solidarisé, pour former la surface d'une sphère.

2. Dispositif (1) dévolu à l'observation et/ou à la manipulation d'objets situés dans une cavité accessible par une ouverture étroite, notamment destiné à l'utilisation en médecine mini-invasive et équipé d'une partie d'insertion (2) en forme de tube ou de tuyau souple, un segment flexible (4) étant ménagé sur ladite partie d'insertion (2), et ladite partie d'insertion (2) renfermant au moins un câble Bowden (5) dédié à l'activation dudit segment flexible (4), sachant qu'un canal de guidage (6), pratiqué dans ladite partie d'insertion (2), est agencé pour recevoir des moyens de liaison optiques, mécaniques et/ou électriques affectés à l'observation et/ou à la manipulation, et sachant qu'il est ménagé un élément de réglage (10) relié audit câble Bowden (5) en vue de l'activation dudit segment flexible (4), ledit élément de réglage (10) étant disposé à une extrémité proximale (8) de la partie d'insertion (2), laquelle extrémité proximale (8) de la partie d'insertion (2) présente une articulation (11) comportant une cuvette d'articulation (13) et un mentonnet d'articulation (12) concordant, l'élément de réglage (10) étant monté dans ladite articulation (11) et ledit élément de réglage (10) étant muni d'une traversée (14), laquelle traversée (14) est agencée en vue de recevoir lesdits moyens de liaison optiques, mécaniques et/ou électriques, et laquelle traversée (14) débouche dans une extrémité proximale (15) du canal de guidage (6) en position d'utilisation de l'élément de réglage (10), **caractérisé par le fait qu'**une zone d'accouplement (20) est ménagée sur l'élément de réglage (10) en vue de la liaison libérable, par concordance de formes, avec une pièce d'accouplement complémentaire (21), un mouvement d'ajustement de ladite pièce d'accouplement complémentaire (21) pouvant être transmis à l'élément de réglage (10), et répercuté sur le segment flexible (4) ; **le fait que** le système d'actionnement (17) est relié à l'élément de réglage (10) par l'intermédiaire d'au moins un câble Bowden secondaire (22), sachant que les câbles Bowden secondaires (22) sont reliés audit élément de réglage (10) à l'aide de la liaison par concordance de formes instaurée entre ledit élément de réglage (10) et la pièce d'accouplement complémentaire (21), et transmettent l'actionnement manuel par l'intermédiaire dudit système d'actionnement (17) ; et **par le fait que**, lors de la dissociation de ladite liaison instaurée entre l'élément de réglage (10) et la pièce d'accouplement complémentaire (21), les câbles Bowden secondaires (22) demeurent reliés à ladite pièce d'accouplement complémentaire (21), tandis que les câbles Bowden primaires (5) demeurent reliés audit élément de réglage (10).

3. Dispositif (1) selon la revendication 1 ou 2, **caractérisé par le fait qu'**une poignée (9), destinée à la partie d'insertion (2), est située à l'extrémité proximale (8) de ladite partie d'insertion (2) ; **par le fait qu'**un système d'actionnement (17) est prévu pour l'actionnement manuel de l'élément de réglage (10) ; et **par le fait que** ledit système d'actionnement (17) est relié à ladite poignée (9) par l'intermédiaire d'un conduit flexible d'activation (19).

4. Dispositif (1) selon l'une des revendications 1 à 3, **caractérisé par le fait que** la cuvette d'articulation (13) est ménagée sur l'élément de réglage (10) et le mentonnet d'articulation (12) est ménagé sur l'extrémité proximale (8) de la partie d'insertion (2) ; ou **par le fait que** ladite cuvette d'articulation (13) est ménagée sur ladite extrémité proximale (8) de la partie d'insertion (2), et ledit mentonnet d'articulation (12) est ménagé sur ledit élément de réglage (10).

5. Dispositif (1) selon la revendication 2 ou l'une des revendications dépendant de cette dernière, **caractérisé par le fait que** la cuvette d'articulation (13) et/ou le mentonnet d'articulation (12) présente(nt) une surface de contact (16), laquelle surface de contact (16) matérialise une région d'une surface cylindrique, sphérique ou elliptique.

6. Dispositif (1) selon l'une des revendications 1 à 5, **caractérisé par le fait qu'**un système d'actionnement (17) est prévu pour l'actionnement manuel de l'élément de réglage (10) ; et/ou **par le fait que** ledit système d'actionnement (17) est relié audit élément de réglage (10) par l'intermédiaire d'un conduit flexible d'activation (19).

7. Dispositif (1) selon l'une des revendications 1 à 6, **caractérisé par le fait que** l'élément de réglage (10) est logé dans une poignée (9) ménagée sur l'extrémité proximale (8) de la partie d'insertion (2).

8. Dispositif (1) selon l'une des revendications 1 à 7, **caractérisé par le fait que** le câble Bowden (5) à présence minimale, et l'élément de réglage (10), et/ou le système d'actionnement (17), sont agencés en vue de l'activation du segment flexible (4) en mode deux brins ou quatre brins ; et/ou **par le fait qu'**il est prévu un système de rappel qui amène ledit élément de réglage (10) à une position normale en cas d'actionnement défectueux.

9. Dispositif (1) selon l'une des revendications 1 à 8, **caractérisé par le fait que** la poignée (9) est munie d'un repère (32) indiquant l'orientation de la partie d'insertion (2) par rapport à des rotations accomplies autour de la direction de l'étendue de celle-ci.

10. Dispositif (1) selon la revendication 1 ou l'une des revendications dépendant de cette dernière, **caractérisé par le fait que** le système d'actionnement (17) est relié à l'élément de réglage (10) par l'intermédiaire d'au moins un câble Bowden secondaire (22) ; et/ou **par le fait que** ledit câble Bowden secondaire (22), à présence minimale dans ledit système d'actionnement (17), vient en prise avec ledit élément de réglage (10), ou avec une pièce d'accouplement complémentaire (21), en au moins un point (25) de venue en prise secondaire dont la distance radiale, vis-à-vis d'un point de rotation (23) de l'articulation (11), diffère de la distance radiale comprise entre un point (24) de venue en prise primaire et ledit point de rotation (23) de l'articulation (11), point (24) de venue en prise primaire auquel le câble Bowden (5) à présence minimale, logé dans la partie d'insertion (2), vient en prise avec ledit élément de réglage (10).

11. Dispositif (1) selon la revendication 2 ou l'une des revendications dépendant de cette dernière, **caractérisé par le fait que** la pièce d'accouplement complémentaire (21) est montée dans une seconde articulation (28) pourvue d'une cuvette d'articulation (27) et d'un mentonnet d'articulation (26) concordant ; et/ou **par le fait que** la cuvette d'articulation (27) et/ou le mentonnet d'articulation (26) de ladite seconde articulation (28) présente(nt) une surface de contact secondaire (29), laquelle surface de contact secondaire (29) matérialise une région d'une surface cylindrique, sphérique ou elliptique, sachant notamment qu'un rayon de courbure de cette surface diffère d'un rayon de courbure de la surface de contact (16) de la première articulation (11).

12. Dispositif (1) selon l'une des revendications 1 à 11, **caractérisé par le fait que** la traversée (14) passe par un point de rotation (23) de l'articulation (11).
